# EUROPEAN PATENT APPLICATION

(11) **EP 2 090 169 A1**
(43) Date of publication of application: **19.08.2009**
(21) Application number: 07790835.8
(22) Date of filing: 17.07.2007
(51) Int. Cl.: A01N 43/42, A01P 3/00, A61K 31/473, A61K 41/00, A61P 31/04

(54) **NUCLEIC ACID-DEGRADING AGENT AND METHOD FOR DEGRADATION OF NUCLEIC ACID**

(30) Priority: 04.12.2006 JP 2006326825
(71) Applicant: Morinaga Milk Industry Co., Ltd., Minato-ku Tokyo 108-8384 (JP)
(72) Inventor: YOSHIDA, Shinichi, Fukuoka-shi Fukuoka 812-8581 (JP); SOEJIMA, Takashi, Zama-shi Kanagawa 228-8583 (JP)
(74) Representative: Bannerman, David Gardner
(86) International application number: PCT/JP2007/064073
(87) International publication number: WO 2008/068923

(57) **Abstract**

The present invention provides an agent for degrading a nucleic acid, which includes ethidium monoazide as an active ingredient, and is useful as an antibacterial agent such as a bactericide or a disinfectant.

## Description

### Technical Field

The present invention relates to an agent for degrading a nucleic acid comprising ethidium monoazide as an active ingredient and to a method of degrading intracellular nucleic acid comprising the steps of adding ethidium monoazide to a cell-containing sample and irradiating the cell-containing sample with visible light to degrade a nucleic acid inside of the cell.

### Background Art

Hitherto, alcohol, cresol, oxidant, and so on have been used as representative medicaments for bactericide/disinfectant. However, they lack in immediate effects for killing bacteria because these agents are protein denaturants. In addition, antibacterial agents including cell wall synthesis inhibitors, protein synthesis inhibitors, nucleic acid metabolic inhibitors, energy metabolic inhibitors and antimetabolite are not enough to anticipate immediate effects because all of them act on proriferation of bacteria to achieve antibacterial activity.

Ethidiummonoazide (3-amino-8-azide-5-ethyl-6-phenyl -phenanthridinium chloride, hereinafter, may be abbreviated as EMA) is an azide compound which has ethidium bromide, synthesized for optical labeling of DNA, as a basic skeleton (Non-patent Document 1). In addition, EMA has been known as a topoisomerase poison to eukaryotic cells (Non-patent Document 2) and used as an agent for cell viability test as well as propidium iodide used for nucleus staining (Non-patent Document 3).

So far, however, the effect of EMA to cleave cellular nucleic acid randomly has not been known in the art.
Non-patent Document 1: Nucleic Acids Res., vol. 5, pages 4891-4903, 1978.
Non-patent Document 2: Biochemistry, No. 50, vol. 36, pages 15884-15891, 1997.
Non-patent Document 3: Appl. Environ. Microbiol., No. 2, vol. 71, pages 1018-1024, 2005.

### Disclosure of the Invention

An Object of the present invention is to provide an agent for degrading a nucleic acid, which is useful as an antibacterial agent including a bactericide or a disinfectant.

The inventors of the present invention have made intensive studies for antibacterial agents, particularly bacteria-killing agents. The inventors have paid their attentions to an agent for degrading a nucleic acid which penetrates a bacterial cell wall and then directly act on bacterial nucleic acid to cleave the nucleic acid, and have made the search for a substance having such an action. As a result, the inventors have completed the present invention by finding that EMA, an azide compound, has an ability of penetrating a living bacterial cell wall and cleaving the nucleic acid.

The first invention according to the present invention to solve the above problems relates to an agent for degrading a nucleic acid comprising ethidium monoazide as an active ingredient.
The second invention according to the present invention to solve the above problems relates to an antibacterial agent comprising the agent for degrading a nucleic acid of the first invention.
The third invention according to the present invention to solve the above problems relates to a method of degrading a nucleic acid in a sample containing the nucleic acid, comprising the steps of adding ethidium monoazide to the sample containing the nucleic acid and irradiating the sample containing the nucleic acid with visible light to degrade the nucleic acid therein.
The fourth invention according to the present invention to solve the above problems relates to a method of degrading a nucleic acid in a cell, comprising the steps of adding ethidium monoazide to a sample containing the cell and irradiating the sample containing the cell with visible light to degrade a nucleic acid inside of the cell.

### Brief Description of the Drawings

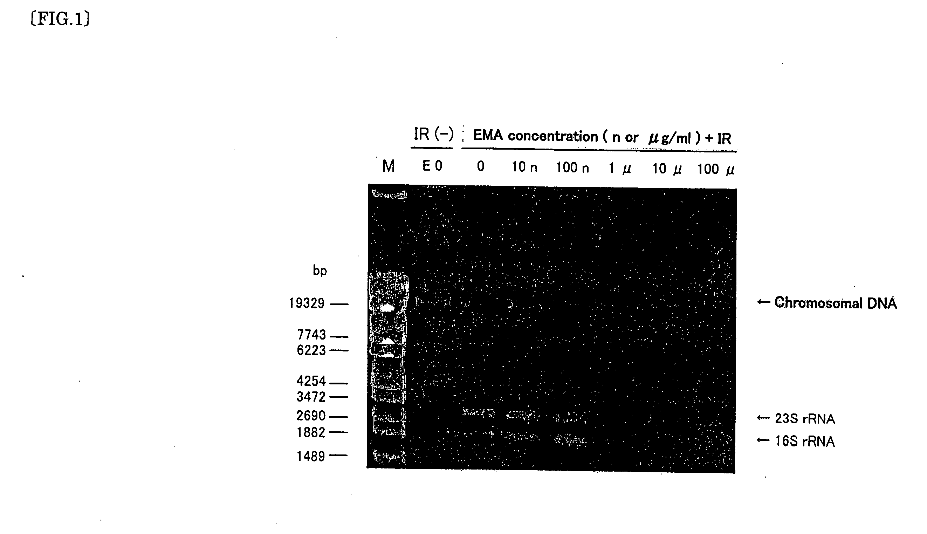

FIG. 1 is an electrophoretic photograph that shows an influence of EMA on chromosomal DNA and rRNA or the like from *E. coli in vitro,* where M indicates a molecular weight marker (λ/EcoT14I digest), IR(-) represents the absence of visible light irradiation, IR represents visible light irradiation (500W halogen bulb, 20 min.), and a numeric value of E0-100n or µ represents the final concentration of EMA (0 to 100 ng/ml or µg/ml).

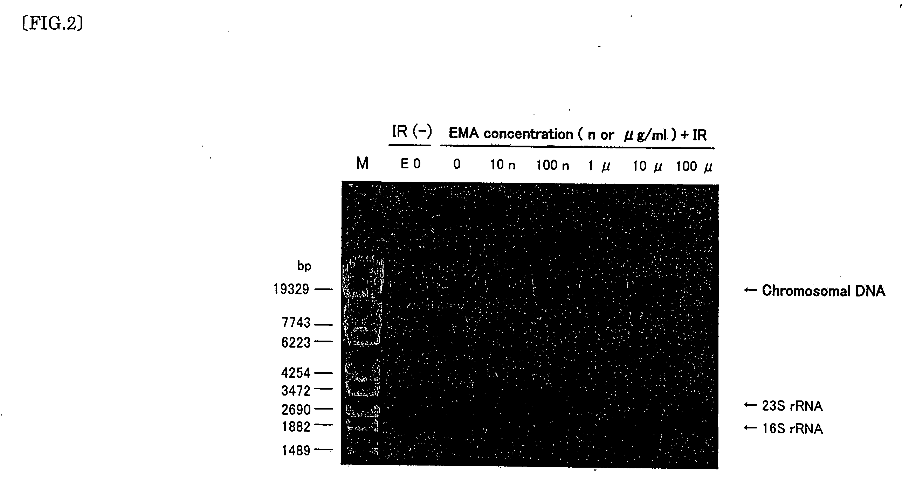

FIG. 2 is an electrophoretic photograph that shows an influence of EMA on chromosomal DNA and rRNA or the like from *E. coli in vivo,* where M indicates a molecular weight marker (λ/EcoT14I digest), IR(-) represents the absence of visible light irradiation, IR represents visible light irradiation (500-W halogen bulb, 20 min.), and a numeric value of E0-100n or µ represents the final concentration of EMA (0 to 100 ng/ml or µg/ml).

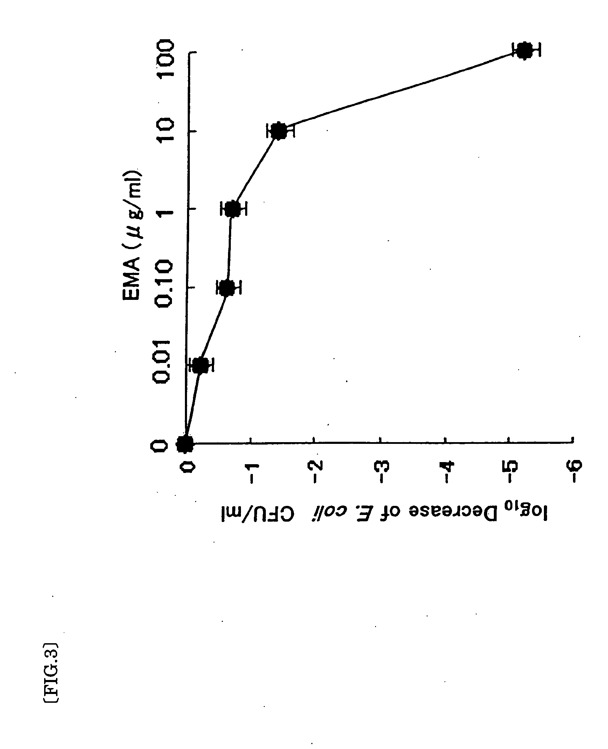

FIG. 3 illustrates the antibacterial effect and dose-response curve of EMA, where the X axis represents the final concentration of EMA (µg/ml) and the Y axis represents a decrease in number of living bacterial cell (CFU/ml) per originally number of living bacterial cells by the common logarithmic in each EMA-treated zone.

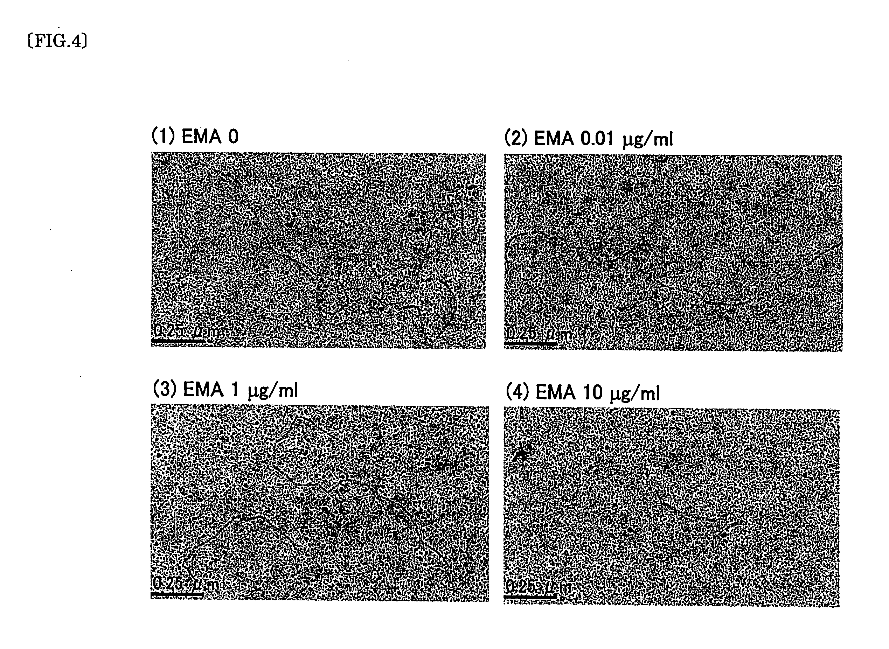

FIG. 4 are photographs representing results of observation with electron microscopy after the treatments of *E. coli* DH5α chromosomal DNA with EMA and visible light irradiation (500W halogen bulb, 20 min.), where the final concentrations of EMA are (1) 0, (2) 0.01 µg/ml, (3) 1 µg/ml, and (4) 10 µg/ml.

### Detailed Description of Preferred Embodiments

Next, preferred embodiments of the present invention will be described in detail. However, the present invention is not limited to the preferred embodiments described below and can be freely modified within the scope of the present invention. Further, expressions in percentage are based on mass unless otherwise noted in this specification.

The agent for degrading a nucleic acid of the present invention has an effect of randomly degrading a nucleic acid by directly acting on an isolated nucleic acid, and also has an effect of randomly cleaving the nucleic acid existing in a sample which contains the nucleic acid using EMA which is an active ingredient and penetrates a sample and directly acts on nucleic acid.
Further, in the present invention, the nucleic acids include DNA and RNA. The nucleic acids to be targeted by the agent for degrading a nucleic acid of the present invention include single-strand DNA, double-strand DNA, single-strand RNA, and double-strand RNA. A sample to be applied in the present invention may contain any of these nucleic acids or may contain two or more of them. In addition, the targets of the agent for degrading a nucleic acid of the present invention include, for example, chromosomal DNA and plasmid DNA, as well as rRNA, mRNA, and tRNA.

Examples of the sample containing nucleic acid include all kinds of biological cells, such as prokaryotic cells (bacteria) and eukaryotic cells (e.g., protists, Eumycetes, plants, and animals), and viruses. Of those, bacteria, Eumycetes, and viruses are preferable.

The agent for degrading a nucleic acid of the present invention, when allowed to act on bacteria, Eumycetes, viruses, or the like, has effects of terminating their growth and killing them by directly degrading the nucleic acid inside of the cells. Therefore, for example, the agent for degrading a nucleic acid of the present invention can be used against environmental microorganism as an antibacterial agent, a bactericide /disinfectant, a virucide, or the like.

Environmental microorganisms to be targeted by the antibacterial agent of the present invention is not specifically limited, but, example thereof include bacteria and Eumycetes. The bacteria include both of gram positive bacteria and gram negative bacteria. The gram positive bacteria include *Staphylococcus* such as *Staphylococcus epidermidis, Streptococcus, Listeria, Bacillus, Mycobacterium*, and *Clostridium*. The gram negative bacteria include *Escherichia* such as *Escherichia coli*, intestinal bacteria such as *Enterobacter, Salmonella, Vibrio, Pseudomonas, Legionella*, and *Campylobacter*.

The Eumycetes to be targeted by the antibacterial agent of the present invention include, but not specifically limited to, *Candida, Aspergillus, Saccharomyces*, and *Penicillium*.

Ethidium monoazide (3-amino-8-azido-5-ethyl-6-phenyl-phenanthridinium chloride: EMA), the active ingredient of the present invention, is a compound represented by the chemical formula (1). The ethidium monoazide used may be of the one commercially available.

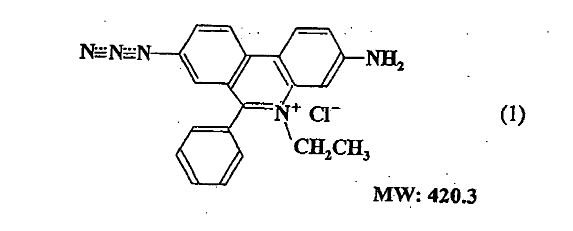

The amount of the agent for degrading a nucleic acid of the present invention to be used may be suitably selected depending on the decision as to either extracellular or intracellular nucleic acid is degraded or depending on the amount of nucleic acid to be degraded. Further, the amount of EMA contained in the agent for degrading a nucleic acid in use may be 1 µg/ml to 1,000 µg/ml, preferably 10 µg/ml to 1,000 µg/ml, particularly preferably 100 µg/ml to 1,000 µg/ml. It is possible to degrade the nucleic acid effectively by allowing the agent for degrading a nucleic acid to act on the target in such a concentration.
The agent for degrading a nucleic acid of the present invention may be a solution or EMA itself. It may be suitably diluted or dissolved when used.

Further, the agent for degrading a nucleic acid of the present invention may be used as an antibacterial agent. Even if the agent for degrading a nucleic acid of the present invention is employed as an antibacterial agent, it acts in the same manner as that of the agent for degrading a nucleic acid.

Further, visible light is irradiated upon degrading the nucleic acid. The wavelength of the visible light to be irradiated is 380 nm to 800 nm, preferably 450 nm to 600 nm. In addition, the visible light may be of mono-wavelength or may be of mixed light whose wavelengths distributed within the above range. The light may include a wavelength other than the above range. In addition, the distance between the optical source and the sample may be suitably selected as long as a sufficient amount of light is irradiated to the targeting sample.

Visible light can be also irradiated by placing the target of the agent for degrading a nucleic acid or the antibacterial agent of the present invention under the irradiation of natural light such as sunlight.

Further, when the agent for degrading a nucleic acid of the present invention is irradiated at an optical strength of 0.5 to 100 W/cm², an effect of degrading the nucleic acid can be exerted sufficiently within about 5 minutes to 1 hour, preferably within about 5 to 30 minutes. For example, when light is irradiated from a 500W halogen bulb at a distance of 20 cm, an effect of degrading the nucleic acid can be exerted sufficiently within about 5 minutes to 1 hour, preferably within about 5 to 30 minutes.

The effects of the agent for degrading a nucleic acid of the present invention can be evaluated by comparing electrophorogram of nucleic acids before and after the addition of the agent for degrading a nucleic acid and the irradiation with visible light irradiation. Further, when the agent for degrading a nucleic acid of the present invention is applied to bacteria, the effect can be also evaluated indirectly by measuring the number of living bacterial cells.

The agent for degrading a nucleic acid of the present invention may be used alone or may be used in combination with other ingredients. For example, the other ingredients include agent for degrading a nucleic acid known in the art, such as exonucleases and endonucleases, e. g. , restriction enzymes, for DNA and RNA. The combination with such agents can further enhance the effect of nucleic acid degradation.

The usage form of the antibacterial agent of the present invention is not particularly limited, but for example, it may be added to a solution or a suitably diluted solution thereof may be sprayed. In addition, the dosage form of the antibacterial agent of the present invention can be suitably selected depending on the application, the usage form thereof, and so on. For example, the dosage forms include, but not specifically limited to, a liquid form, a granular form, and a tablet form.

Further, the antibacterial agent of the present invention may be used alone or in combination with other ingredients. The other ingredients may be antibacterial agents or bactericides, and examples thereof include antibiotics, alcohols such as ethanol and isopropyl alcohol, oxidants such as phenol, cresol, halogen compounds (e.g., chlorine and iodine), and peroxides (e.g., ozone and hydrogen peroxide) and heavy metal compounds. The combination with such ingredients can further enhance the antibacterial effect.

The antibacterial agent of the present invention may be, for example, preferably used for disinfection of instruments and so on and also disinfection of wall surfaces, floors, and so on. In addition, the antibacterial agent of the present invention may be sprayed in the indoor space, thereby it is very useful in sterilization of bacteria (pathogenic *Escherichia coli, Mycobacterium tuberculosis, botulinum, Bacillus anthracis*, and so on) having high risks of severity when they infect humans.

The antibacterial agent of the present invention may directly act on intracellular nucleic acid to degrade the nucleic acid. It is not almost necessary to consider a problem of the resistance of bacteria, the antibacterial agent of the present invention has an excellent antibacterial activity and a wide antibacterial spectrum in comparison with known antibacterial agents.

Next, the present invention will be further described in detail with reference to examples, but the invention is not limited to the examples described below.

### Example 1

This example was carried out for investigation of an influence of EMA on nucleic acid such as *E. coli* chromosomal DNA, rRNA, *in vitro.*
(1) Test Method
1ml living bacterial suspension of 1.0 x 10⁶ CFU/ml of *E. coli*/DH5α strain was subjected to centrifugation under a cool condition. After removal of the supernatant, the resulting pellet was added with 0.5 ml of 10mM Tris-HCl buffer solution (pH 8.0) and further added with 10 µl of 1250U/ml protease K solution and 200 µl of 10% SDS solution, followed by overnight bacteriolysis at 50°C.

Subsequently, each of the treated solution was divided into two equal volumes and dispensed into two 2ml micro tubes, respectively. Each of them was added with 0.5 ml of saturated phenol solution, then gently mixed for 15 minutes, and then added with 0.5 ml of chloroform, followed by gently mixing for 5 minutes. After that, the mixture was centrifuged at 6, 000 x g at 4°C for 10 minutes. An aqueous phase, an upper layer, was transferred into an another 2ml microtube and then added with 70 µl of 3M sodium acetate (pH 5.2) and 1.21 ml of 99.5% cold ethanol, followed by gentle mixing. Subsequently, the mixture was centrifuged at 15,000 x g at 4°C for 10 minutes and the supernatant thereof was then removed. After that, 0.4 ml of 70% cold ethanol was added, thereby washing a pellet (precipitation) (hereinafter, the above series of operations may be abbreviated as a phenol/chloroform extraction). Subsequently, the pellet was added with 0.5 ml of 10mM Tris-HCl buffer (pH 8.0) containing 1mM EDTA/2Na solution (TE buffer) and then left standing overnight at 4°C, thereby dissolving the nucleic acid. The concentration of the purified nucleic acid solution was determined based on the absorbance at UV260 nm (50 µg/ml of the nucleic acid was defined as OD = 1, cell length = 1 cm : OD₂₆₀).

The nucleic acid solution thus prepared was adjusted to 175 ng/µl with sterile water and 4µl aliquot of the nucleic acid solution was then added to each of microtubes. Subsequently, 4 µl of aqueous EMA solutions (0, 0.02, 0.2, 2, 20, and 200 µg/ml) were respectively added to the microtubes and then left standing at 4°C for 1 hour under light interception. After that, the sample was irradiated for 20 minutes with visible light from a 500W halogen bulb (FLOOD PRF 100V 500W; Iwasaki Electric Co., Ltd., Tokyo) at a distance of 20 cm from the sample. The whole volume of the sample was electrophoresed on a 0.7% agarose gel. λ-EcoT14 I digest (manufactured by Takara Bio Inc.) was used as a molecular weight marker. The gel after the electrophoresis was stained with 1 µg/ml of an ethidium bromide solution and then irradiated with UV at 254 nm using an UV trans-illuminator. The resulting image was recorded on the Polaroid film 667. An untreated nucleic acid solution (EMA: 0 µg/ml, without irradiation of visible light) was used as a control and then similarly electrophoresed in the same way.

(2) Test results
The results of the test are shown in FIG. 1. Consequently, the intensity of the band originated from the chromosomal DNA of approximately 19,329 bps was gradually decreased from 100 ng/ml to 1 µg/ml of EMA concentration and significantly disappeared at 10 µg/ml of EMA concentration. Thus, it was confirmed that the EMA degraded the chromosomal DNA in the nucleic acid isolated from the living bacteria (*E. coli*). Further, it was confirmed that the band intensity of rRNA (16SrRNA and 23SrRNA) was decreased with 1 µg/ml of EMA and disappeared with 10 µg/ml or more of EMA. Thus, it was also confirmed that rRNA was degraded.

### Example 2

This example was carried out for investigating an influence of EMA on nucleic acid such as *E. coli* chromosomal DNA, rRNA, *in vivo*.
(1) Test method
EMA was dissolved in sterile water to prepare 1000µg/ml EMA solution. The solution was filtrated for sterilization through a 0.2µm filter (Minisart-plus; manufactured by Sartorius AG). The EMA solution was added so that the final concentration of EMA become 0 (no addition), 0.01, 0.1, 1, 10, and 100 µg/ml with respect to 1 ml of 1.0 x 10⁶CFU/ml living bacterial suspension of *E. coli*/DH5α strain, followed by leaving standing at 4°C for 1 hour.

Subsequently, at a distance of 20 cm from the above living bacterial suspension on ice, the sample was irradiated for 20 minutes with visible light from a 500W halogen bulb (FLOOD PRF 100V 500W; Iwasaki Electric Co., Ltd., Tokyo). The sample was subjected to centrifugation at 15, 000 x g at 4°C for 10 minutes and the supernatant was then removed to eliminate a product generated by the reaction of water with the visible light irradiation product of EMA (hydroxyamino ethidium), which could not covalently bind to nucleic acid. The pellet was added with 0.5 ml of 10mM Tris-HCl buffer (pH 8.0) and then added with 10 µl of 1250U/ml protease K solution and 200 µl of 10% SDS solution. The bacteriolytic operation was carried out overnight at 50°C.

Each of the treated solution was divided into two equal volumes and dispensed into two 2ml micro tubes, respectively. Each of them was added with 0.5 ml of saturated phenol solution, then gently mixed for 15 minutes, and then added with 0. 5 ml of chloroform, followed by gently mixing for 5 minutes. After that, the mixture was centrifuged at 6,000 x g at 4°C for 10 minutes. An aqueous phase, an upper layer, was transferred into an another 2ml microtube and then added with 70 µl of 3M sodium acetate (pH 5.2) and 1.21 ml of 99.5% cold ethanol, followed by gentle mixing. Subsequently, the mixture was centrifuged at 15,000 x g at 4°C for 10 minutes and the supernatant thereof was then removed. After that, 0.4 ml of 70% cold ethanol was added, thereby washing a pellet (precipitation) (hereinafter, the above series of operations may be abbreviated as a phenol/chloroform extraction). Subsequently, the pellet was added with 0.5 ml of 10mM Tris-HCl buffer containing 1mM EDTA/2Na (TE buffer) and then left standing overnight at 4°C, thereby dissolving the nucleic acid. The concentration of the purified nucleic acid solution was determined based on the absorbance at UV260 nm (50 µg/ml of the nucleic acid was defined as OD = 1, cell length = 1 cm : OD₂₆₀). The purity of the purified nucliec acid was calculated by dividing OD₂₆₀ with OD₂₈₀.

Each of the nucleic acid solutions was prepared at 175 ng/µl and 4µl of each was then electrophoresed on 0.7% agarose gel. λ-EcoT14 I digest (manufactured by Takara Bio Inc.) was used as a molecular weight marker. The gel after the electrophoresis was stained with 1 µg/ml of ethidium bromide solution and then irradiated with UV at 254 nm using an UV trans-illuminator. The resulting image was recorded on the Polaroid film 667. An untreated living bacterial suspension of *E. coli* (EMA: 0 µg/ml, without irradiation of visible light) was subjected to nucleic acid extraction and used as a control.

(2) Test results
The results of the test are shown in FIG. 2. Consequently, the intensity of the band originated from the chromosomal DNA of approximately 19,329 bps was gradually decreased with 10 µg/ml of EMA and significantly disappeared with 100 µg/ml of EMA. Thus, it was confirmed that the EMA could degrade the chromosomal DNA in the nucleic acid existing inside of the living bacteria (*E. coli*).
Further, it was confirmed that the band intensity of rRNA (16SrRNA and 23SrRNA) was decreased with 10 µg/ml of EMA. Thus, it was also confirmed that rRNA of living bacteria (*E. coli*) could be degraded.

### Example 3

This example was carried out for investigating an antibacterial effect of EMA on the living bacteria.
(1) Test method
Ethidium monoazide (EMA) was dissolved in sterile water to prepare 1000µg/ml EMA solution. The solution was filtrated through a 0.2µm sterile filter (Minisart-plus; manufactured by Sartorius AG). The EMA solution was added so that the final concentration of EMA become 0 (no addition), 0.01, 0.1, 1, 10, and 100 µg/ml, respectively with respect to 1 ml of 1. 0 x 10⁶CFU/ml living bacterial suspension of *E. coli*/DH5α strain, followed by left standing at 4°C for 1 hour under light interception.

Subsequently, at a distant of 20 cm from the above living bacterial suspension on ice, the sample was irradiated for 20 minutes with visible light from a 500W halogen bulb (FLOOD PRF 100V 500W; Iwasaki Electric Co., Ltd., Tokyo). The sample was subjected to centrifugation at 15, 000 x g at 4°C for 10 minutes and the supernatant was then removed to eliminate a product generated by the reaction of water with the visible light irradiation product of EMA (hydroxyamino ethidium), which could not covalently bind to nucleic acid. The pellet was added with an equal amount of physiological saline solution and then serially diluted, followed by 24hour incubation at 37°C using an L-plate agar culture medium to determine the number of living bacterial cells.

(2) Test results
The antibacterial effect of EMA was illustrated as a dose-response curve in FIG. 3. As a result, it was confirmed as follows: when EMA at a concentration of 10 µg/ml was reacted with *E. coli,* the number of living bacterial cells was decreased in the order of 10² CFU/ml in comparison with the original number of the living bacterial cells. When EMA at a concentration of 100 µg/ml was reacted with *E. coli,* the number of living bacterial cells was decreased in the order of 10⁵ CFU/ml in comparison with the original number of the living bacterial cells.
Thus, it was found that EMA has an antibacterial effect on *E. coli* (living cells).

### Example 4

This example was carried out for observing an effect of EMA on *E. coli* chromosomal DNA *in vitro* under an electron microscopy.
(1) Test method
Nucleic acid was extracted in a manner similar to Example 1 as described above. The nucleic acid was then dissolved in 9 ml of sterile water. A nucleic acid solution thus prepared was gently loaded on the top of 32ml sucrose density gradient (16 ml of 10% sucrose solution and 16 ml of 40% sucrose solution were used) and then subjected to ultracentrifugation at 26,000 rpm at 20°C for 18 hours with a swing rotor (manufactured by Hitachi Koki Co., Ltd. : RPS-27-2). After the centrifugation, a small hole was opened in the bottom of the sucrose density gradient solution and then fractionated every 1 ml.

Afterthat, for each fraction, 3Msodiumacetatesolution (pH 5.2) was added so as to be 10% (vol/vol). Then, 2-fold volume of 99.5% ethanol was added. Subsequently, the fraction was subj ected to centrifugation at 15,000 x g at 4°C for 10 minutes to recover the pellet. Then, the pellet was washed with 70% ethanol and then dissolved in 100 µl of sterile water.

Among the samples obtained from the respective fraction solutions, a sample only containing a long chromosomal DNA with 48 kbp in agarose electrophoresis was further diluted with sterile water so as to be a DNA concentration of 175 ng/µl Then 4 µl of each aqueous EMA solution (0, 0.02, 2, and 20 µg/ml) was added to 4 µl of the DNA solution and then left standing at 4°C for 1 hour under light interception. Subsequently, the sample on ice was irradiated for 20 minutes with visible light from the 500W halogen bulb described above.

The DNA solution (8 µl) after irradiation of the above visible light was diluted five-folds (32 µl addition) with sterile water. 1 µ1 of 0.02% cytochrome C solution was added to 5 µ1 of 8% formaldehyde solution, and the total amount thereof was mixed with 40 µ1 of the DNA solution, followed by leaving standing for 10 minutes. Subsequently, a chytochrome C membrane was collected by tweezers, and a dehydration treatment with 90% ethanol was carried out. Subsequently, the staining was carried out with a solution of 0.5 mM uranium acetate / 0.5 mM hydrochloric acid / 90% ethanol, followed by dehydration treatment with 90% ethanol and isopentane.
For electro microscopic observation, a shadowing was carried out using platinum/palladium powder and a photograph was then taken by an electron microscopy (manufactured by JEOL Ltd., JEOL T-2000 EX).

(2) Test results The results of the test are shown in FIG. 4. FIG. 4 shows the results of observation with electron microscopy after the treatment of *E.coli*. chromosomal DNA with EMA in a concentration of 0 to 10 µg/ml and visible light irradiation (500W halogen bulb, for 20 minutes). As a result, an effect of cleaving the chromosomal DNA was not observed after reacting with 0 to 0.01 µg/ml of EMA. However, it was confirmed that the reaction with EMA in a concentration of 1 µg/ml or more caused the cleaving phenomenon of chromosomal DNA. Such results correspond to those in Example 1 and the cleaving phenomena of the chromosomal DNA with EMA was visually confirmed.
Industrial Applicability

The agent for degrading a nucleic acid of the present invention has an ability to pass through the cell wall of a living bacteria and is capable of randomly cleaving chromosomal DNA, RNA, or the like of the bacteria. Therefore, in the fields of bacteriology and biochemistry, it is very useful as an antibacterial agent, particularly a bactericide/disinfectant against environmental microorganisms. In addition, the agent for degrading a nucleic acid of the present invention can be preferably used in the field of research.

## Claims

1. An agent for degrading a nucleic acid comprising ethidiummonoazide as an active ingredient.

2. An antibacterial agent comprising the agent for degrading a nucleic acid of claim 1.

3. A method of degrading a nucleic acid in a sample containing the nucleic acid, comprising the steps of adding ethidium monoazide to the sample containing the nucleic acid and irradiating the sample containing the nucleic acid with visible light to degrade the nucleic acid therein.

4. A method of degrading a nucleic acid in a cell, comprising the steps of adding ethidium monoazide to a sample containing the cell and irradiating the sample containing the cell with visible light to degrade a nucleic acid inside of the cell.
